# EUROPEAN PATENT APPLICATION

(11) **EP 3 205 347 A1**
(43) Date of publication of application: **16.08.2017**
(21) Application number: 15849024.3
(22) Date of filing: 06.10.2015
(51) Int. Cl.: A61K 38/00, A23L 33/10, A61P 7/02, A61P 9/00, A61P 13/12, A61P 29/00, A61P 37/02, C07K 14/47

(54) **WHITE BLOOD CELL EXTRACELLULAR TRAP FORMATION INHIBITOR**

(30) Priority: 08.10.2014 JP 2014207415
(71) Applicant: Keio University, Tokyo 108-8345 (JP); NRL Pharma, Inc., Takatsu-ku Kawasaki-shi, Kanagawa 213-0012 (JP); Kawakami, Hiroshi, Kawagoe-shi, Saitama 350-1142 (JP)
(72) Inventor: KAWAKAMI, Hiroshi, Kawagoe-shi Saitama 350-1142 (JP); HIRAHASHI, Junichi, Tokyo 160-8582 (JP); OKUBO, Koshu, Tokyo 160-8582 (JP); KAGAYA, Shinji, Kawasaki-shi Kanagawa 213-0012 (JP)
(74) Representative: HGF Limited
(86) International application number: PCT/JP2015/078857
(87) International publication number: WO 2016/056665

(57) **Abstract**

An object of the present invention is to provide a novel drug inhibiting extracellular trap formation in leukocytes. The present invention provides an inhibitor of extracellular trap formation in leukocytes containing a lactoferrin fragment, and a composition containing lactoferrin for treating a disease related to the extracellular trap formation in leukocytes.

## Description

### Technical Field

The present invention relates to an inhibitor of extracellular trap formation in leukocytes containing a peptide fragment similar to lactoferrin as an active ingredient, and a composition for treating a disease related to extracellular trap formation in leukocytes. Further, the present invention relates to a method, using the inhibitor or the composition, for treating a disease related to the extracellular trap formation in leukocytes.

### Background Art

NETs (neutrophil extracellular traps) are extracellular structures that exhibit microbial activity through activation of neutrophils by bacterial infection to make the form of segmented neutrophils and the distribution of chromatin unclear, followed by bread-down of nuclear membranes to allow cytoplasms and granule components to be mixedly present in a chromatin structure, releasing network structures upon rupture of cell membrane, and capturing bacteria, fungi, parasites and viruses. A NET is constituted mainly by a DNA, and histone 3 (H3) and elastase significantly affect its activity. The formation of NETs results in local collection of antimicrobial molecules efficiently killing microorganisms. When the NETs are formed, a cell death (NETosis) of the neutrophils is caused, and although the molecular mechanism thereof has not yet been elucidated, it is caused by stimulation of TNF-α, PMA, LPS, IL-8 and the like, and the cell death caused here is different from conventional necrosis and apoptosis causing caspase activation and DNA fragmentation. When neutrophils are stimulated with LPS or PMA, autophagy occurs, and at the same time, reactive oxygen is generated. As a result, nuclear membrane breakdown, decondensation of chromatin and citrullination of histone occur, which causes NETosis (Non Patent Literatures 1 and 2).

It has been reported that following the NET formation, a large amount of proteins, particularly antimicrobial proteins contained in azurophilic granules and secondary granules are secreted by degranulation, and proteins involved in cell structures are also secreted (Non Patent Literature 2). These proteins are also designated as NET component proteins, and neutrophil elastase, histone, myeloperoxidase, F-actin, lactoferrin, matrix metalloprotease 9, LL37, cathepsin G, BPI, proteinase 3, calprotectin, azurocidin, lysozyme C, defensin, catalase and the like have been reported as such proteins (Non Patent Literature 3).

Lactoferrin is contained in secondary granules of a neutrophil, and when the neutrophil forms a NET to be ultimately degranulated, the lactoferrin is released around (Non Patent Literature 1).

Lactoferrin is well preserved in mammals, and there is no large species difference in its function. Besides, lactoferrin currently attracts attention for the physiological activity thereof as a protein of a constituent component of milk, and is commercially available. It has been, however, reported that milk does not show an effect of inhibiting NET formation in a bovine neutrophil (Non Patent Literature 4).

The NETs are also involved in enlargement/growth of a thrombus, and when the NETs are released, histone contained therein has a function to aggregate platelets, and hence, a platelet thrombus is formed based on the NETs. In addition, neutrophil elastase and cathepsin G contained in the NETs decompose a tissue factor pathway inhibitor and accelerate a blood coagulation reaction. Owing to these actions, the NETs play a role to topically confine microorganisms and the like (Non Patent Literature 5).

The inherent action of the NETs is to capture and topically confine foreign microorganisms such as gram-positive bacteria, gram-negative bacteria and fungi for sterilization. The NETs are often formed in infectious diseases because of this action, and it has also been reported that the NETs are formed also in the absence of foreign microorganisms according as an infectious disease or the like becomes chronic. In systemic lupus erythematosus (SLE), that is, one of chronic and refractory autoimmune diseases, it is known that an autoantibody to a self DNA or a related protein is formed so as to cause inflammation in tissue or organ. As a characteristic finding of SLE, a large number of neutrophils are present in a damaged region. It is known that a serum of an SLE patient contains antimicrobial peptide LL37 and a DNA contained in the NETs (Non Patent Literatures 6 and 7). They are identified as an autoantigen by B cells, and hence an autoantibody is produced. Moreover, NETosis is more likely to occur in neutrophils of an SLE patient than in neutrophils of a heathy individual (Non Patent Literature 6). It is regarded that such factors induce chronic inflammation. Furthermore, with respect to anti-neutrophil cytoplasmic antibody (ANCA)-associated vasculitis, that is, a disease caused by an autoantibody, it has been reported that an IgG fraction of a serum of a patient of this disease has NET formation ability about twice as high as an IgG fraction of a healthy individual (Non Patent Literatures 8 and 9).

It is merely recently, specifically, after a report of Brinkman et al., in 2004 (Non Patent Literature 1) that the relationship between the NET formation and diseases has started to attract attention, and in the future, other diseases caused by the NET formation will be revealed. As described above, the NET formation plays a significant role in protection of a living body against infection, and there may be a case where inhibition of the NET formation is necessary for improving some of disease states.

As a substance inhibiting the NET formation, it has been reported that streptococci of gram-positive cocci such as Streptococcus pneumoniae and Staphylococcus aureus express DNase outside fungus bodies for decomposing a released DNA network. Further, as substances inhibiting the production of an antibody to histone and a superoxide, diphenyleneiodonium chloride (DPI) and catalase have been reported (Non Patent Literature 10).

In addition, it is known that myeloperoxidase (MPO) activity affects the NETosis (Non Patent Literature 11), and that a phenomenon of cell death caused by the NET formation in a neutrophil, namely, NETosis, is a different process from apoptosis and necrosis (Non Patent Literature 10).

In one conventional technique (Patent Literature 1), a milk-derived basic protein fraction is used as an active ingredient for preventing autoimmune diseases such as type I diabetes and rheumatoid arthritis, but the contents of this literature are specialized in adjustment of immune cells mainly of lymphocytes and inhibition of inflammatory cytokine, and does not relate to a function to inhibit the NET formation. Up to the present, no drug for treating a disease derived from the NET formation has been reported.

### [Citation List]

### [Patent Literature]

Patent Literature 1: Japanese Patent Laid-Open No. 2008-189637

### [Non Patent Literature]

Non Patent Literature 1: Volker Brinkmann et al., 2004, Science, 303: 1532-1535
Non Patent Literature 2: Q. Remijsen et al., 2011, Cell Death and Differentiation, 18: 581-588
Non Patent Literature 3: Maren von Koeckritz-Blickwede et al., 2008, Blood, 111: 3070-3080
Non Patent Literature 4: John D. Lippolis et al., 2006, Veterinary Immunology and Immunopathology, 113: 248-255
Non Patent Literature 5: Fuchs, T. A. et al., 2010, Proc Natl Acad Sci USA, 107: 15880-15885
Non Patent Literature 6: Garcia-Romo, G. S. et al., 2011 Sci Transl Med, 3: 73ra20
Non Patent Literature 7: Lande, R. et al., 2011 Sci Transl Med, 3: 73ra19
Non Patent Literature 8: Kessenbrock, K. et al., 2009, Nat Med, 6: 623-625
Non Patent Literature 9: Bosch, X., 2009, J Am Soc Nephrol, 8: 1654-1656
Non Patent Literature 10: Tobias A. Fuchs et al., 2007, J Cell Biol, 176: 231-241
Non Patent Literature 11: K. Akong-Moore et al., 2012, PLOS ONE, 7: e42984
Non Patent Literature 12: Makoto Naito et al., 2010, Journal of Analytical Bio-Science, 33: 329-338

### Summary of Invention

An object of the present invention is to provide a fundamental therapeutic agent for a disease developed by extracellular trap formation in leukocytes, in particular, a safe and effective therapeutic agent and a treatment method suitable for long-term maintenance of remission (recurrence inhibition).

The present inventors have made earnest studies to solve the aforementioned problem, resulting in finding that a lactoferrin fragment remarkably shows an effect of inhibiting NET formation and is capable of a fundamental treatment of NET-related diseases.

Specifically, the present invention provides the following:
[1] An inhibitor of extracellular trap formation in leukocytes comprising, as an active ingredient, a peptide containing an amino acid sequence shown as:

   X1-K-C-X2-X3-X4-Q-X5-X6-X7-X8-X9

   wherein
   X1 is S, T, F, A, K, E or Q;
   X2 is R, F, S, Y, A or L;
   X3 is Q, R, K or M;
   X4 is W or F;
   X5 is R, W, V, N, S or E;
   X6 is R, N, E, K or M;
   X7 is M, L or I;
   X8 is R, K or N; and
   X9 is K or R.
[2] The inhibitor of extracellular trap formation according to [1] above, wherein X1 is S or T in the amino acid sequence.
[3] The inhibitor of extracellular trap formation according to [1] above, wherein X2 is R, F, S, Y or A in the amino acid sequence.
[4] The inhibitor of extracellular trap formation according to [1] above, wherein X3 is Q, R or K in the amino acid sequence.
[5] The inhibitor of extracellular trap formation according to [1] above, wherein X4 is W in the amino acid sequence.
[6] The inhibitor of extracellular trap formation according to [1] above, wherein X5 is R in the amino acid sequence.
[7] The inhibitor of extracellular trap formation according to [1] above, wherein X6 is R or N in the amino acid sequence.
[8] The inhibitor of extracellular trap formation according to [1] above, wherein X7 is M or L in the amino acid sequence.
[9] The inhibitor of extracellular trap formation according to [1] above, wherein X8 is R or K in the amino acid sequence.
[10] The inhibitor of extracellular trap formation according to [1] above, wherein X9 is K in the amino acid sequence.
[11] The inhibitor of extracellular trap formation according to [1] above, wherein X1 is S in the amino acid sequence.
[12] The inhibitor of extracellular trap formation according to [1] above, wherein X3 is Q in the amino acid sequence.
[13] The inhibitor of extracellular trap formation according to [1] above, wherein X7 is M in the amino acid sequence.
[14] The inhibitor of extracellular trap formation according to [1] above, wherein X8 is R in the amino acid sequence.
[15] The inhibitor of extracellular trap formation according to [1] above, further comprising a residue X0 on the N-terminal side of the residue X1, in which X0 is A, M, W, G, S, E or Y.
[16] The inhibitor of extracellular trap formation according to [15] above, wherein X0 is A.
[17] The inhibitor of extracellular trap formation according to any one of [1] to [16] above, further comprising, on the C-terminal side of the residue X9, an amino acid sequence of:

   X10-X11-X12-P-X13-X14-X15-C-X16-X17-X18-X19-X20

   wherein X10 is V, L, T or A, preferably X10 is V or L, and more preferably X10 is V;
   X11 is G, R or N, preferably X11 is G or R, and more preferably X11 is G;
   X12 is G, A or V, preferably X12 is G or A, and more preferably X12 is G;
   X13 is S, P, I or L, more preferably X13 is S or P, and more preferably X13 is S;
   X14 is V, I, L or M, preferably X14 is V or I, and more preferably X14 is V;
   X15 is S, T, F or R, preferably X15 is S or T, and more preferably X15 is S;
   X16 is I, V, T or A, preferably X16 is I or V, and more preferably X16 is I;
   X17 is R or K, and preferably X17 is R;
   X18 is R or K, and preferably X18 is R;
   X19 is A, T, D, S or Y, and preferably X19 is A or T; and
   X20 is S or F, and preferably X20 is S.
[18] The inhibitor of extracellular trap formation according to any one of [1] to [14] above, wherein the peptide contains any one amino acid sequence selected from the group consisting of amino acid sequences shown as SEQ ID NOS: 6 to 16.
[19] The inhibitor of extracellular trap formation according to any one of [1] to [18] above, prepared in such a manner that a dose of the peptide is 0.001 to 10 g/kg/day.
[20] The inhibitor of extracellular trap formation according to any one of [1] to [19] above, wherein the leukocyte is any one selected from the group consisting of a neutrophil, an eosinophil, a basophil, a monocyte, a macrophage and a mast cell.
[21] The inhibitor of extracellular trap formation according to any one of [1] to [20] above, wherein the leukocyte is a neutrophil.
[22] A composition for treating a disease related to extracellular trap formation in leukocytes, comprising, as an active ingredient, a peptide containing an amino acid sequence shown as:

   X1-K-C-X2-X3-X4-Q-X5-X6-X7-X8-X9

   wherein X1 is S, T, F, A, K, E or Q;
   X2 is R, F, S, Y, A or L;
   X3 is Q, R, K or M;
   X4 is W or F;
   X5 is R, W, V, N, S or E;
   X6 is R, N, E, K or M;
   X7 is M, L or I;
   X8 is R, K or N; and
   X9 is K or R.
[23] The composition according to [22] above, prepared in such a manner that a dose of the peptide is 0.001 to 10 g/kg/day.
[24] The composition according to [22] or [23] above, wherein the leukocyte is any one selected from the group consisting of a neutrophil, an eosinophil, a basophil, a monocyte, a macrophage and a mast cell.
[25] The composition according to [22] or [23] above, wherein the leukocyte is a neutrophil.
[26] The composition according to any one of [22] to [25] above, wherein the disease is any one selected from the group consisting of vasculitis syndrome, ANCA-associated vasculitis, systemic lupus erythematosus, local Shwartzman reaction, acute kidney injury (AKI) accompanied by ischemia-reperfusion injury and disseminated intravascular coagulation.
[27] The composition according to any one of [22] to [26] above, in the form of food.
[28] The composition according to any one of [22] to [26] above, in the form of an injection.
[29] The composition according to any one of [22] to [26] above, to be administered orally.
[30] The composition according to any one of [22] to [26] above, to be administered transdermally.

The present invention provides a treatment method for a disease caused by extracellular trap formation in leukocytes in which a few adverse reactions occur. This method also has an advantage, due to the few adverse reactions, that it can be reliably employed for a wide range of patients and patient candidates.

The inhibitor and the composition of the present invention can be widely used for a variety of subjects including immunocompromised subjects such as aged persons and cancer-bearing patients, and subjects having previous histories of infectious complications or tuberculosis. In addition, the present invention provides a therapeutic agent and a treatment method for a disease related to the extracellular trap formation in leukocytes with which a few adverse reactions occur even if used for a long period of time. The therapeutic agent is useful particularly when the agent needs to be used for a long period of time in the treatment of the diseases, or as a recurrence inhibitor for a subject having been remitted from an acute symptom, or when the diseases have become chronic.

### Brief Description of Drawings

[Figure 1] Figure 1 is a diagram illustrating an alignment of amino acid sequences of lactoferricin derived from various animals.
[Figure 2] Figure 2 is a graph illustrating NET formation inhibiting ability obtained by a human lactoferrin fragment.
[Figure 3] Figure 3 is a graph illustrating NET formation inhibiting ability obtained by a human lactoferrin fragment.

### Description of Embodiments

The present invention will now be described in detail. The following embodiments are merely illustrative of the present invention but not intended to limit the present invention to these embodiments alone. The present invention can be practiced in a variety of embodiments within the scope thereof.

It is noted that all literatures and patent literatures including patent laid-open publications and patent publications cited herein are incorporated herein by reference. Further, this application embraces the contents of the specification and drawings of Japanese patent application (Japanese Patent Application No. 2014-207415) filed on October 8, 2014, based on which the present application claims the benefit of priority.

Herein, a base sequence is described with the 5'-terminal disposed on the left side and the 3'-terminal disposed on the right side unless otherwise mentioned. Similarly, an amino acid sequence is described with the amino terminal (the N-terminal) disposed on the left side and the carboxy terminal (the C-terminal) disposed on the right side.

Moreover, each of amino acids is expressed using a one-letter code or a three-letter code as follows:
[Table 1]

**Table 1**

| Name of Amino Acid | One-letter Code | Three-letter Code |
|---|---|---|
| Alanine | A | Ala |
| Arginine | R | Arg |
| Asparagine | N | Asn |
| Aspartic Acid | D | Asp |
| Cysteine | C | Cys |
| Glutamine | Q | Gln |
| Glutamic Acid | E | Glu |
| Glycine | G | Gly |
| Histidine | H | His |
| Isoleucine | I | Ile |
| Leucine | L | Leu |
| Lysine | K | Lys |
| Methionine | M | Met |
| Phenylalanine | F | Phe |
| Proline | P | Pro |
| Serine | S | Ser |
| Threonine | T | Thr |
| Tryptophan | W | Trp |
| Tyrosine | Y | Tyr |
| Valine | V | Val |

### 1. Inhibitor of Extracellular Trap Formation in Leukocyte

The present invention provides, as a first embodiment, an inhibitor of extracellular trap formation in leukocytes (hereinafter referred to as the "inhibitor of the present invention") containing a lactoferrin fragment.

Extracellular traps have been reported to be formed in various leukocytes, and for example, reported to be formed in a neutrophil (Brinkmann, V. et al., Science 2004; 303: 1532-1535), a basophil (Yousefi, S. et al., Nat Med 2008; 14: 949-953), a mast cell (von Koeckritz-Blickwede M. et al., Blood 2008; 111: 3070-3080), a monocyte (Webster, S. J. et al., J Immunol 2010; 185: 2968-2979): for example, a macrophage (Chow, O. A. et al., Cell Host & Microbe, Volume 8, Issue 5, 445-454, 18 November 2010)), and the like.

It has been reported that the extracellular traps formed in these leukocytes have a common characteristic of releasing a fiber component containing a DNA and a granule protein as principal components (Simon, D. et al., Allergy 68 (2013) 409-416).

On the contrary, the inhibitor of the present invention can inhibit the release of the fiber component by concentrating and/or condensing the fiber component. Accordingly, when the inhibitor of the present invention is used, a fiber component released, at the time of extracellular trap formation, not only from a neutrophil used in examples but also from other leukocytes (such as a basophil, a mast cell and a monocyte (for example, a macrophage) can be concentrated and/or condensed, so that the extracellular trap formation in these leukocytes can be inhibited.

The lactoferrin fragment used as the active ingredient of the inhibitor of the present invention is not especially limited as long as it is derived from milk of a mammal (such as a monkey, a bovine, a goat, a sheep, a human, a camel, a horse, a dog, a cat, a mouse or a rat), is preferably a fragment of lactoferrin derived from mammal milk ingestible by a human (such as milk of a bovine, a goat, a sheep or a human), and is more preferably a fragment of human milk-derived lactoferrin. Alternatively, the lactoferrin and the fragment thereof may be derived from a neutrophil of any of the aforementioned mammals.

Amino acid sequences of lactoferrin derived from various mammals are known (see Table 2 below).
[Table 2]

**Table 2**

| Lactoferrin Derivation | Genbank Accession No. | SEQ ID NO: |
|---|---|---|
| Human | NP_001186078.1 | 1 |
| Bovine | NP_851341.1 | 2 |
| Sheep | ACT76166.1 | 3 |
| Goat | AAA97958.1 | 4 |
| Horse | CAA09407.1 | 5 |

The lactoferrin fragment is not especially limited as long as it is obtained by fragmenting full-length lactoferrin, and may be an acidic fraction containing a large number of acidic amino acid residues or a basic fraction containing a large number of basic amino acid residues. In one embodiment, the lactoferrin fragment is a fragment obtained by pepsin hydrolysis of full-length lactoferrin and is preferably a basic fraction, and in another embodiment, the lactoferrin fragment is lactoferricin.

Here, the term acidic amino acid residue refers to an amino acid residue selected from aspartic acid (D) and glutamic acid (E).

In addition, the term basic amino acid residue refers to an amino acid residue selected from arginine (R), histidine (H) and lysine (K).

In one embodiment, a peptide used in the inhibitor of the present invention is a lactoferrin fragment containing an amino acid sequence shown as:

X1-K-C-X2-X3-X4-Q-X5-X6-X7-X8-X9

wherein X1 is S, T, F, A, K, E or Q;
X2 is R, F, S, Y, A or L;
X3 is Q, R, K or M;
X4 is W or F;
X5 is R, W, V, N, S or E;
X6 is R, N, E, K or M;
X7 is M, L or I;
X8 is R, K or N; and
X9 is K or R.

Hereinafter, the amino acid sequence shown as "X1-K-C-X2-X3-X4-Q-X5-X6-X7-X8-X9" is designated as the "generic sequence". The peptide used in the inhibitor of the present invention, namely, the lactoferrin fragment, is hereinafter referred to as the "LF fragment of the present invention".

In the generic sequence, X1 is preferably S or T, and more preferably X1 is S.

X2 is preferably R, F, S, Y or A.

X3 is preferably Q, R or K, and more preferably X3 is Q.

X4 is preferably W.

X5 is preferably R.

X6 is preferably R or N.

X7 is preferably M or L, and more preferably X7 is M.

X8 is preferably R or K, and more preferably X8 is R.

X9 is preferably K.

In some cases, the LF fragment of the present invention may contain "X1-K-C-X2-X3-X4-Q-X5-X6" (9-mer) or "X1-K-C-X2" (4-mer), or may consist of these amino acid sequences.

Specifically, the LF fragment of the present invention may be a fragment containing or consisting of any one amino acid sequence selected from the group consisting of:

"X1-K-C" (3-mer);

"X1-K-C-X2" (4-mer);

"X1-K-C-X2-X3" (5-mer);

"X1-K-C-X2-X3-X4" (6-mer);

"X1-K-C-X2-X3-X4-Q" (7-mer);

"X1-K-C-X2-X3-X4-Q-X5" (8-mer);

"X1-K-C-X2-X3-X4-Q-X5-X6" (9-mer);

"X1-K-C-X2-X3-X4-Q-X5-X6-X7" (10-mer);

"X1-K-C-X2-X3-X4-Q-X5-X6-X7-X8" (11-mer);

and

"X1-K-C-X2-X3-X4-Q-X5-X6-X7-X8-X9" (12-mer),

and in either case, the LF fragment has activity of inhibiting the extracellular trap formation in leukocytes (which activity will be described later).

In the each aforementioned 3-mer to 9-mer amino acid sequences, X1 to X9 are defined as described above.

In one embodiment, the LF fragment of the present invention may further contain a residue X0 on the N-terminal side of the residue X1 of the generic sequence. In this case, the LF fragment of the present invention contains the following amino acid sequence:

X1-K-C-X2-X3-X4-Q-X5-X6-X7-X8-X9

wherein X1 to X9 are defined as described above, and
X0 is A, M, W, G, S, E or Y, and preferably X0 is A.

Alternatively, the LF fragment of the present invention may further contain, on the C-terminal side of the residue X9 of the generic sequence, an amino acid sequence, X10-X11-X12-P-X13-X14-X15-C-X16-X17-X18-X19-X20. In this case, the LF fragment of the present invention contains the following amino acid sequence:

X1-K-C-X2-X3-X4-Q-X5-X6-X7-X8-X9-X10-X11-X12-P-X13- X14-X15-C-X16-X17-X18-X19-X20

wherein X1 to X9 are defined as described above;
X10 is V, L, T or A, preferably X10 is V or L, and more preferably X10 is V;
X11 is G, R or N, preferably X11 is G or R, and more preferably X11 is G;
X12 is G, A or V, preferably X12 G or A, and more preferably X12 G;
X13 is S, P, I or L, preferably X13 is S or P, and more preferably X13 is S;
X14 is V, I, L or M, preferably X14 is V or I, and more preferably X14 is V;
X15 is S, T, F or R, preferably X15 is S or T, and more preferably X15 is S;
X16 is I, V, T or A, preferably X16 is I or V, and more preferably X16 is I;
X17 is R or K, and preferably X17 is R;
X18 is R or K, and preferably X18 is R;
X19 is A, T, D, S or Y, and preferably X19 is A or T; and
X20 is S or F, and preferably X20 is S.

In another embodiment, the LF fragment of the present invention may contain another amino acid sequence X on one of or both of the N-terminal side of the residue X0 and the C-terminal side of the residue X20 of the aforementioned generic sequence. In this case, the LF fragment of the present invention contains the following amino acid sequence:

X-X0-X1-K-C-X2-X3-X4-Q-X5-X6-X7-X8-X9-X10-X11-X12-P- X13-X14-X15-C-X16-X17-X18-X19-X20-X

wherein X0 to X20 are defined as described above; and
X is an amino acid sequence consisting of optional 1 to 16 amino acid residues.

In a still another embodiment, the LF fragment of the present invention consists of any one amino acid sequence selected from the group consisting of amino acid sequences shown as SEQ ID NOS: 6 to 16. Here, SEQ ID NOS: 6 to 16 show amino acid sequences of lactoferricin derived from various animals (see Table 3 below).
[Table 3]

**Table 3 Amino Acid Sequences of Lactoferricin derived from Various Animals**

| Species | SEQ ID NO: |
|---|---|
| Bovine | 6 |
| Human | 7 |
| Monkey | 8 |
| Goat | 9 |
| Sheep | 10 |
| Horse | 11 |
| Camel | 12 |
| Dog | 13 |
| Mouse | 14 |
| Pig | 15 |
| Rat | 16 |

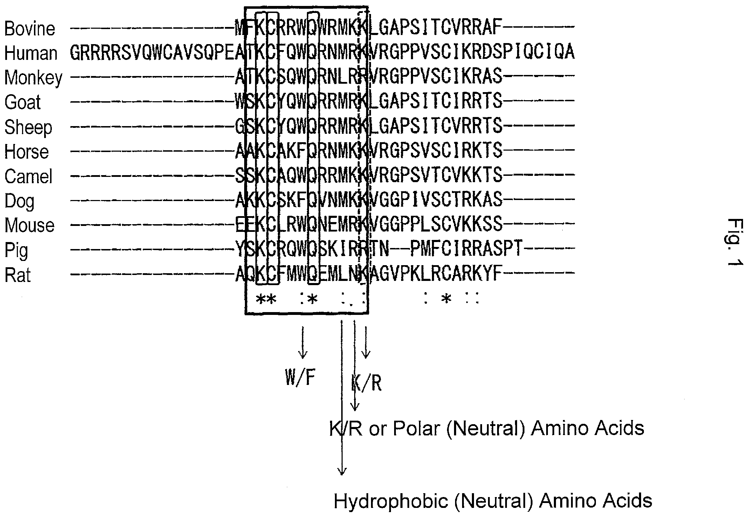

As illustrated in Figure 1, the amino acid sequences shown as SEQ ID NOS: 6 to 16 can be comprehensively represented by the aforementioned generic sequence.

The lactoferrin fragment has the activity of inhibiting the extracellular trap formation in leukocytes as described in examples below.

Here, in the "activity of inhibiting the extracellular trap formation in a leukocyte", the leukocyte is derived from a living being in which an extracellular trap in the leukocyte is formed, preferably derived from a vertebrate, and more preferably derived from a mammal. Examples of the mammal include a monkey, a bovine, a goat, a sheep, a human, a camel, a horse, a dog, a cat, a mouse and a rat, and the mammal is preferably a human.

Furthermore, the leukocyte is derived from any of the above, and at the same time, is preferably any one selected from the group consisting of a neutrophil, an eosinophil, a basophil, a monocyte, a macrophage and a mast cell, is more preferably any one selected from the group consisting of a neutrophil, a basophil, a monocyte, a macrophage and a mast cell, and is further preferably a neutrophil.

The activity of inhibiting the extracellular trap formation in the leukocyte can be confirmed by confirming that the extracellular trap formation is reduced in the presence of the LF fragment of the present invention upon microscopic observation on a culture system where the leukocyte is cultured in the presence or the absence of the lactoferrin fragment, namely, the LF fragment of the present invention.

In the above-described test for confirming the activity, the leukocyte is preferably subjected to a treatment with an extracellular trap formation accelerator (such as paramethoxyamphetamine (PMA) or lipopolysaccharide (LPS)) before adding the LF fragment of the present invention.

Alternatively, when a culture supernatant of the above-described culture system is collected and a DNA concentration in the supernatant is measured, the activity of inhibiting the extracellular trap formation in the leukocyte can be confirmed. Such a DNA concentration is mainly derived from a DNA released into the culture supernatant at the time of the extracellular trap formation.

If the extracellular trap formation is inhibited by the LF fragment of the present invention, the DNA concentration in the supernatant is lower than that obtained in the absence of the LF fragment of the present invention.

Also in this case, the leukocyte is preferably subjected to the treatment with an extracellular trap formation accelerator (such as paramethoxyamphetamine (PMA) or lipopolysaccharide (LPS) before adding the LF fragment of the present invention.

The DNA concentration can be easily measured by using a commercially available kit (Picogreen dsDNA assay reagent (P11496 Invitrogen)).

The culture conditions for each leukocyte and the method for confirming the extracellular trap can be determined by referring to the following: For a neutrophil (Brinkmann, V. et al., Science 2004; 303: 1532-1535), A. K. Gupta. FEBS letters 2010; 584: 3193-3197, D. J. Novo, Antimicrob Agents Chemother. 2000; 44(4): 827-34); for a basophil (Yousefi, S. et al., Nat Med 2008; 14: 949-953), for a mast cell (von Kockritz-Blickwede M. et al., Blood 2008; 111: 3070-3080), for a monocyte (Webster, S. J. et al., J Immunol 2010; 185: 2968-2979): for example, for a macrophage (Chow, O. A. et al., Cell Host & Microbe, Volume 8, Issue 5, 445-454, 18 November 2010)).

In addition, the LF fragment of the present invention may be modified by a compound. For example, the LF fragment of the present invention may be a modified protein bonded to polyethylene glycol (Japanese Patent No. 4195486, Japanese Patent No. 4261531, and International Publication No. WO2009/113743), or a fusion protein fused to another protein or a fragment thereof (such as a protein IgG or albumin stable in blood, or a fragment thereof) (Japanese Patent Laid-Open No. 2012-98085).

### 2. Treatment Method

The extracellular trap formation in leukocytes can be effectively inhibited by using the inhibitor of the present invention. In other words, a disease related to the extracellular trap formation in leukocytes can be treated by using the inhibitor of the present invention (a method for which treatment is hereinafter referred to as the "treatment method of the present invention").

The term "treatment" used herein means improvement of a symptom of a human or other mammals in general. Further, the term "improvement" refers to that the degree of a disease is lowered and not worsened as compared with a case where the LF fragment of the present invention is not administered, and also embraces meaning of prevention.

In this case, a treatment subject (a patient) is a living being suffering from or a living being having a risk of a disease related to the extracellular trap formation in leukocytes, is preferably a vertebrate, and is more preferably a mammal. Examples of the mammal include mammals selected from the group consisting of a human, a bovine, a horse, a goat, a sheep, a dog and a cat, and the mammal is more preferably a human.

In the treatment method of the present invention, the "disease related to the extracellular trap formation in leukocytes" is not especially limited as long as it is a disease in which increase of the extracellular trap formation in leukocytes is observed in a body of a patient.

Examples of such a disease include vasculitis syndrome, ANCA-associated vasculitis (such as Wegener's granulomatosis, microscopic polyangiitis, or allergic granulomatous angiitis), acute kidney injury (AKI) accompanied by ischemia-reperfusion injury, systemic lupus erythematosus (SLE), appendicitis, Aspergillus infection, pneumonia, pneumococcal infection, necrotizing fasciitis, streptococcal infection, sepsis, preeclampsia, Crohn's disease, schistosomiasis, periodontitis, tuberculosis, mastitis, malaria, cystic fibrosis, and thrombotic diseases such as deep vein thrombosis (von Bruhl, M. L. et al., J Exp Med. 2012 Apr 9; 209(4): 819-35), myocardial infarction (de Boer, O. J., Thromb Haemost. 2013 Feb; 109(2): 290-7), tumor-associated thrombosis (Demers, M., Proc Natl Acad Sci USA. 2012 Aug 7; 109(32): 13076-81) and disseminated intravascular coagulation (DIC) (Tobias, A. et al, blood, 29 September 2011, vol. 118, no. 13, p. 3708-3714) (Non Patent Literatures 2 and 12). The vasculitis syndrome is classified into large-vessel vasculitis, medium-vessel vasculitis and small-vessel vasculitis depending on the size of the affected vessel. The NET-related diseases such as ANCA-associated vasculitis and SLE described above are classified as the small-vessel vasculitis, but DIC frequently occurs in combination with severity increase of polyarteritis nodosa, that is, a medium-vessel vasculitis ("Junkankibyo no Shindan to Chiryo ni kansuru Gaidorain" (Guideline for Diagnosis and Treatment of Cardiovascular Diseases) (2006-2007 Joint Research Group Report)), sepsis and solid cancer. In these diseases, cytotoxicity caused by the formation of extracellular trap in leukocytes (such as NETs) causes vascular endothelial dysfunction, and therefore, dysfunction of an organ occurs. Moreover, in the aforementioned thrombotic diseases, the formation of extracellular trap in leukocytes (such as NETs) triggers acceleration of cascade of thrombus formation. It is presumed that the LF fragment of the present invention prevents the vascular endothelial dysfunction by inhibiting the formation and release/diffusion of the extracellular trap in leukocytes (such as NETs), and furthermore, shows an organ protecting action by inhibiting the cascade of thrombus formation, and hence shows a therapeutic effect against the aforementioned diseases.

The disease to be treated by the treatment method of the present invention is preferably vasculitis syndrome, ANCA-associated vasculitis (such as Wegener's granulomatosis, microscopic polyangiitis, or allergic granulomatous angiitis), systemic lupus erythematosus, local Schwatzman reaction or acute kidney injury (AKI) accompanied by ischemia-reperfusion injury, and is more preferably microscopic polyangiitis accompanied by increase of an antibody value of MPO-ANCA (myeloperoxidase specific anti-neutrophil cytoplasmic antibody) in blood, allergic granulomatous angiitis, or disseminated intravascular coagulation (DIC).

Incidentally, although a treatment of an autoimmune disease such as type I diabetes or rheumatoid arthritis using lactoferrin has been also reported (Patent Literature 1), the extracellular trap formation in leukocytes is not regarded as the principal cause of the autoimmune disease such as type I diabetes or rheumatoid arthritis, and hence, there is a high possibility that lactoferrin works not through the extracellular trap formation in the treatment described in this report.

Specifically, in the present invention, it can be said that the lactoferrin fragment shows a therapeutic effect against the above-described diseases through a completely novel mechanism, namely, a mechanism for inhibiting the extracellular trap formation in leukocytes.

In the treatment of SLE and diseases caused due to ANCA, steroid, immunosuppressant or the like is used, but such a treatment is accompanied by physical burden of a patient (such as an adverse reaction) and unavoidable discomfort to the patient, and in addition, has a problem that there is a high risk of inducing another disease (infectious disease).

On the contrary, according to the present invention, since a lactoferrin fragment conventionally contained in food is used as the active ingredient, it is advantageous that a few adverse reactions are caused, that it does not impart discomfort to a patient, and that there is a low risk of inducing another disease.

### 3. Composition

As another embodiment, the present invention provides a composition that contains the LF fragment of the present invention for treating a disease related to the extracellular trap formation in leukocytes (hereinafter referred to as the "composition of the present invention").

Here, the term "composition" means one containing an additive such as a carrier used in preparation of the active ingredient (the lactoferrin fragment of the present invention) useful in the present invention.

In the composition of the present invention, the "LF fragment of the present invention" and the "disease related to the extracellular trap formation in leukocytes" are defined as described above.

An administration route of the composition of the present invention is not especially limited as long as it is a generally employed route, and specific examples include oral, sublingual, nasal, transpulmonary, transgastrointestinal and transdermal routes, eye drop, intravenous injection, subcutaneous injection, intramuscular injection, intraperitoneal injection, local injection, and surgical transplantation, and the oral administration and the intravenous administration are preferred.

The composition of the present invention may be in the form of a solid formulation such as a capsule, a tablet or a powder, or may be in the form of a liquid formulation such as a solution, a suspension or an emulsion, or in the form of a semi-liquid formulation such as an ointment, a cream or a paste.

If the oral administration is employed in the present invention, the composition is preferably in the form of a solid formulation. Alternatively, if the injection administration is employed, the composition is in the form of a solid formulation including one obtained by freeze drying, or a liquid formulation. If the transdermal administration is employed, the composition may be prepared in the form of a liquid formulation (including a spray formulation) such as a solution, a suspension or an emulsion, or in the form of a semi-liquid formulation such as an ointment, a cream or a paste, or may be prepared in the form of a poultice formulation.

The LF fragment of the present invention may be added to food or feed, so as to be eaten as food or feed by a human or a non-human subject animal. A method for producing such food or feed is also known to those skilled in the art. Further, it can be formulated in the form of a solution formulation to be used as an injection.

Alternatively, the LF fragment of the present invention may be added to a nutritional supplement, a diet or the like directly or after being prepared into a formulation.

The LF fragment of the present invention may be used alone, or may be mixed with another pharmaceutically acceptable component.

For example, if it is prepared as an orally administered composition in the form of a powder, a granule, a tablet, a capsule or the like, it is formulated by a conventional method using starch, lactose, sucrose, mannite, carboxymethyl cellulose, corn starch, inorganic salt or the like. In this type of composition, a coating agent, a binder, a disintegrator, a surfactant, a lubricant, a flow accelerator, a colorant, a flavor or the like can be appropriately used in addition to the aforementioned excipient.

The composition of the present invention can contain a therapeutically effective amount of the active ingredient of the lactoferrin fragment. Here, the term "therapeutically effective amount" refers to an amount of the active ingredient with which the symptom of a disease related to the extracellular trap formation in leukocytes is reduced or not worsen as compared with a case where the lactoferrin fragment is not administered, and embraces meaning of an amount effective for prevention.

For example, for the oral administration, the therapeutically effective amount can be 0.001 to 10 g/kg/day, 0.005 to 10g/kg/day, 0.01 to 10 g/kg/day or 0.01 to 5 g/kg/day. If it is administered to a human, the therapeutically effective amount is, in general, 10 mg to 15,000 mg, 10 mg to 12,000 mg, 10 mg to 10,000 mg, 20 mg to 10,000 mg, 20 mg to 8,000 mg, 30 mg to 8,000 mg or 30 mg to 6,000 mg per day. Alternatively, for the transdermal administration, the therapeutically effective amount can be 0.001 to 10 g/kg/day, 0.005 to 10g/kg/day, 0.01 to 10 g/kg/day or 0.01 to 5 g/kg/day. If it is administered to a human, the therapeutically effective amount is, in general, 10 mg to 15,000 mg, 10 mg to 12,000 mg, 10 mg to 10,000 mg, 20 mg to 10,000 mg, 20 mg to 8,000 mg, 30 mg to 8,000 mg or 30 mg to 6,000 mg per day. Such a dose per day can be administered at one time or dividedly to a patient requiring a treatment of a disease related to the extracellular trap formation in leukocytes.

It is noted that the dose and administration frequency of the composition of the present invention vary depending on various factors such as the species, the weight, the sex, and the age of a subject, tumor disease staging, and the administration route, and those skilled in the art including a doctor, a veterinarian, a dentist and a pharmacist can determine the dose in consideration of these factors.

The therapeutically effective amounts, the doses and the administration frequencies mentioned above are described merely as representative values, and a therapeutic effect can be sufficiently obtained in some cases even if the amount and the like are larger or smaller than those mentioned above. Accordingly, the therapeutically effective amount, the dose and the administration frequency of the composition of the present invention also embrace those larger or smaller than the therapeutically effective amounts, doses and administration frequencies described above.

The present invention will now be described in detail with reference to examples, and it is noted that the present invention is not limited to embodiments described in the examples.

### Examples

The present invention will now be described in detail with reference to Examples, and it is noted that the present invention is not limited to embodiments described in the Examples.

Effect of Inhibiting NET Formation Stimulation of Healthy Individual's Peripheral Blood Neutrophil by Pretreatment with Lactoferrin-derived Peptide Fragment

### 1. Method for Isolating Human Neutrophils

For each round of experiment, 15 ml to 20 ml of peripheral blood was collected from a healthy individual by using an EDTA-containing syringe (with a needle of 18 to 22G). After collecting the blood, in a 15 ml conical tube charged with 3 ml of Mono-Poly Resolving Medium (Cat No. DSBN100, DS Pharma Biochemical Co., Ltd.), 3.5 ml of the whole blood was gently layered over a lower layer of Mono-Poly Resolving Medium. The resultant 15 ml conical tube was centrifuged with a swing centrifuge at room temperature (15 to 30°C) at 400 x g for 20 minutes, and the conical tube was then gently taken out. An uppermost brown plasma layer and a lymphocyte/monocyte layer thereunder were removed with an aspirator or the like. In addition, a transparent layer under the lymphocyte/monocyte layer was removed as much as possible. A pale pink layer under the transparent layer was taken out with a Pasteur pipette, and transferred, for washing neutrophils, to a test tube charged with phosphate buffered saline (PBS(-); 137 mM of sodium chloride, 2.7 mM of potassium chloride, 8.1 mM of disodium hydrogen phosphate dodecahydrate, and 1.47 mM of potassium dihydrogen phosphate). The test tube thus holding the neutrophils was centrifuged at room temperature (15 to 30°C) at 200 x g for 10 minutes. The test tube was taken out, and a supernatant was discarded. To the neutrophils held in the test tube, sterile water at 4°C was added, and the resultant was allowed to stand still on ice for 30 seconds for hemolyzing erythrocytes mixed therein. After the hemolysis, 45 ml of PBS(-) was added thereto, and the resultant was centrifuged at 200 x g for 10 minutes. The test tube was taken out, a supernatant was discarded, and a precipitate was suspended in a medium DMEM + 2% human serum Alb (Serum human albumin; Product No. A9080 Sigma + 4mM L-glutamine) to be stored at 8°C until use. The number of neutrophils that could be separated was 1 x 10⁶/ml to 1 x 10⁷/ml, and the purity obtained by visual counting in a sample having been subjected to cytospin and Giemsa staining was 95 to 98%.

### 2. Pretreatment Method (NET Formation Inhibition) for Neutrophil and NETosis (NET Formation) Inducing Method

About 1 hour after the isolation of the neutrophils described in "1. Method for Isolating Human Neutrophil" above, the neutrophils were subjected to a pretreatment. In an 8 well-µ slide (Cat. No. ib 80826 Ibidi (registered trademark)), the neutrophils were seeded into 400 µl of medium DMEM + 2% human serum Alb (Serum human albumin; Product No. A9080 Sigma + 4 mM L-glutamine) at about 1.0 x 10⁵ cells/well, and a lactoferrin peptide fragment was added thereto for performing the pretreatment at room temperature for 30 minutes.

### 3. Method for Measuring DNA Concentration in Culture Supernatant

A culture supernatant was collected, centrifuged at 200 x g for 10 minutes, and the thus obtained supernatant was transferred to a fresh microcentrifuge tube. The amount of DNA was measured by using Picogreen dsDNA assay reagent (P11496 Invitrogen) in accordance with the accompanying protocol. When the human lactoferrin was subjected to the pretreatment, the DNA concentration in the culture supernatant was reduced in a concentration-dependent manner, and thus, the release of the DNA due to the NET was inhibited.

### 4. Method for Preparing Basic Peptide and Acidic Peptide from Bovine Lactoferrin

Bovine lactoferrin was dissolved in sterile water to a final concentration of 5% (w/v), and pH of the resultant aqueous solution was lowered to 2.5 using hydrochloric acid. Thereafter, pepsin (E/S = 1/50) was added to the thus obtained lactoferrin solution, and the resultant was allowed to stand still at 37°C for 5 minutes. Hydrolysis of the lactoferrin was stopped by controlling the pH of the aqueous solution to 7.0 using sodium hydroxide. A basic peptide of the lactoferrin was isolated and purified using AKTA Explorer 10S (GE Healthcare Japan). A 20 mM sodium phosphate buffer (pH = 7.0) to which 2 M sodium chloride had been added was used for removing impurities and an undigested portion of the lactoferrin and then a 20 mM sodium phosphate buffer (pH = 7.0) to which 2 M ammonium chloride had been added was used for eluting a basic peptide (SEQ ID NO: 6) of the lactoferrin.

Acidic peptides of the lactoferrin (peptides respectively containing amino acid residues at positions 316 to 332 of amino acid sequences shown as NCBI Protein Database Accession Nos. 3IAZ_A, 3IB1_A, 3IB0_A, 3IB2_A, 4OQO_A and 4OQO_B) were chemically synthesized in accordance with the F-MOC method (9-fluorenylmethyloxycarbonyl group) using ABI 433A peptide Synthesizer (Life Technologies Corporation). The amino acid sequence of each of the prepared peptides was analyzed by a protein sequencer PPSQ-21A (Shimadzu Corporation).

### 5. Preparation of Basic Peptides

Preparation of the other basic peptides (SEQ ID NOS: 6 and 7) was entrusted to PH Japan Co., Ltd.

### 6. Results

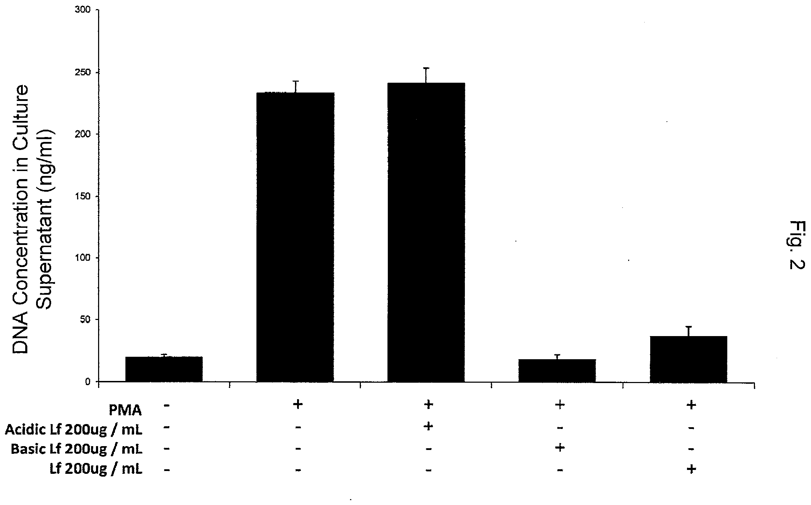

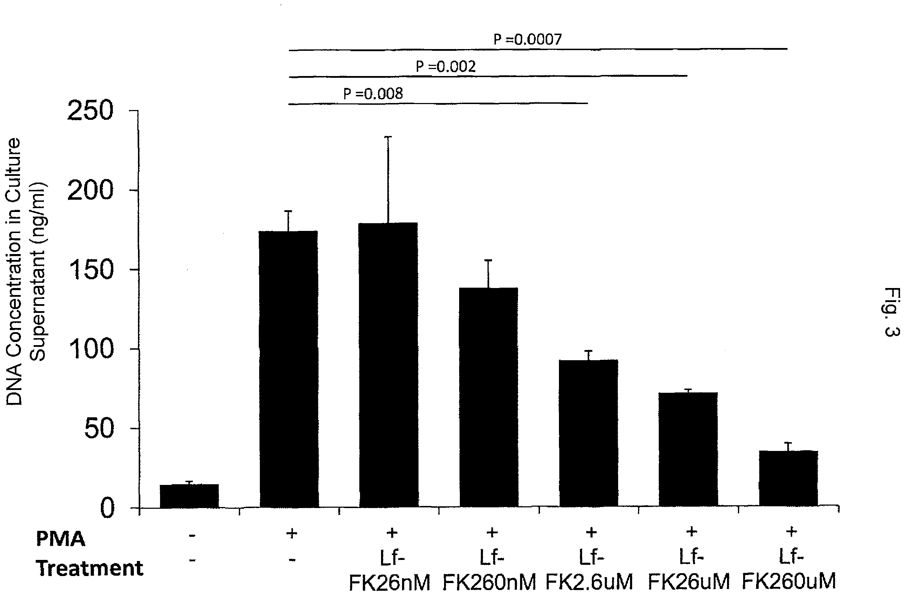

The acidic peptides derived from the lactoferrin did not inhibit the NET. The basic peptide (SEQ ID NO: 6) was found to have the inhibiting effect in a region encoding lactoferricin (Figure 2). In addition, peptide regions encoding lactoferricin (SEQ ID NOS: 6 and 7) inhibited the NET formation in a volume-dependent manner (Figure 3).

### Industrial Applicability

The present invention can provide a treatment method with a few adverse reactions for a disease caused by extracellular trap formation in leukocytes. Further, due to the few adverse reactions, the method has an advantage that it can be reliably employed for a wide range of patients and patient candidates.

In addition, since it has been confirmed that the inhibiting ability for the extracellular trap formation in leukocytes of the present invention is obtained in a non-autoimmune disease model, it can be said that the lactoferrin fragment of the present invention shows a treatment action through a completely novel mechanism of action different from the mechanism of action precedently reported (Patent Literature 1).

## Claims

1. An inhibitor of extracellular trap formation in leukocytes comprising, as an active ingredient, a peptide containing an amino acid sequence shown as:
X1-K-C-X2-X3-X4-Q-X5-X6-X7-X8-X9
wherein
X1 is S, T, F, A, K, E or Q;
X2 is R, F, S, Y, A or L;
X3 is Q, R, K or M;
X4 is W or F;
X5 is R, W, V, N, S or E;
X6 is R, N, E, K or M;
X7 is M, L or I;
X8 is R, K or N; and
X9 is K or R.

2. The inhibitor of extracellular trap formation according to claim 1, wherein X1 is S or T in the amino acid sequence.

3. The inhibitor of extracellular trap formation according to claim 1, wherein X2 is R, F, S, Y or A in the amino acid sequence.

4. The inhibitor of extracellular trap formation according to claim 1, wherein X3 is Q, R or K in the amino acid sequence.

5. The inhibitor of extracellular trap formation according to claim 1, wherein X4 is W in the amino acid sequence.

6. The inhibitor of extracellular trap formation according to claim 1, wherein X5 is R in the amino acid sequence.

7. The inhibitor of extracellular trap formation according to claim 1, wherein X6 is R or N in the amino acid sequence.

8. The inhibitor of extracellular trap formation according to claim 1, wherein X7 is M or L in the amino acid sequence.

9. The inhibitor of extracellular trap formation according to claim 1, wherein X8 is R or K in the amino acid sequence.

10. The inhibitor of extracellular trap formation according to claim 1, wherein X9 is K in the amino acid sequence.

11. The inhibitor of extracellular trap formation according to claim 1, wherein X1 is S in the amino acid sequence.

12. The inhibitor of extracellular trap formation according to claim 1, wherein X3 is Q in the amino acid sequence.

13. The inhibitor of extracellular trap formation according to claim 1, wherein X7 is M in the amino acid sequence.

14. The inhibitor of extracellular trap formation according to claim 1, wherein X8 is R in the amino acid sequence.

15. The inhibitor of extracellular trap formation according to claim 1, further comprising a residue X0 on the N-terminal side of the residue X1, wherein X0 is A, M, W, G, S, E or Y.

16. The inhibitor of extracellular trap formation according to claim 15, wherein X0 is A.

17. The inhibitor of extracellular trap formation according to any one of claims 1 to 16, further comprising, on the C-terminal side of the residue X9, an amino acid sequence of:
X10-X11-X12-P-X13-X14-X15-C-X16-X17-X18-X19-X20
wherein
X10 is V, L, T or A;
X11 is G, R or N;
X12 is G, A or V;
X13 is S, P, I or L;
X14 is V, I, L or M;
X15 is S, T, F or R;
X16 is I, V, T or A;
X17 is R or K;
X18 is R or K;
X19 is A, T, D, S or Y; and
X20 is S or F.

18. The inhibitor of extracellular trap formation according to any one of claims 1 to 14, wherein the peptide contains any one amino acid sequence selected from the group consisting of amino acid sequences shown as SEQ ID NOS: 6 to 16.

19. The inhibitor of extracellular trap formation according to any one of claims 1 to 18, prepared in such a manner that a dose of the peptide is 0.001 to 10 g/kg/day.

20. The inhibitor of extracellular trap formation according to any one of claims 1 to 19, wherein the leukocyte is any one selected from the group consisting of a neutrophil, an eosinophil, a basophil, a monocyte, a macrophage and a mast cell.

21. The inhibitor of extracellular trap formation according to any one of claims 1 to 20, wherein the leukocyte is a neutrophil.

22. A composition for treating a disease related to extracellular trap formation in leukocytes, comprising, as an active ingredient, a peptide containing an amino acid sequence shown as:
X1-K-C-X2-X3-X4-Q-X5-X6-X7-X8-X9
wherein
1 is S, T, F, A, K, E or Q;
X2 is R, F, S, Y, A or L;
X3 is Q, R, K or M;
X4 is W or F;
X5 is R, W, V, N, S or E;
X6 is R, N, E, K or M;
X7 is M, L or I;
X8 is R, K or N; and
X9 is K or R.

23. The composition according to claim 22, prepared in such a manner that a dose of the peptide is 0.001 to 10 g/kg/day.

24. The composition according to claim 22 or 23, wherein the leukocyte is any one selected from the group consisting of a neutrophil, an eosinophil, a basophil, a monocyte, a macrophage and a mast cell.

25. The composition according to claim 22 or 23, wherein the leukocyte is a neutrophil.

26. The composition according to any one of claims 22 to 25, wherein the disease is any one selected from the group consisting of vasculitis syndrome, ANCA-associated vasculitis, systemic lupus erythematosus, local Shwartzman reaction, acute kidney injury (AKI) accompanied by ischemia-reperfusion injury and disseminated intravascular coagulation.

27. The composition according to any one of claims 22 to 26, in the form of food.

28. The composition according to any one of claims 22 to 26, in the form of an injection.

29. The composition according to any one of claims 22 to 26, to be administered orally.

30. The composition according to any one of claims 22 to 26, to be administered transdermally.

31. The composition according to any one of claims 22 to 30, wherein X1 is S or T in the amino acid sequence.

32. The composition according to any one of claims 22 to 30, wherein X2 is R, F, S, Y or A in the amino acid sequence.

33. The composition according to any one of claims 22 to 30, wherein X3 is Q, R or K in the amino acid sequence.

34. The composition according to any one of claims 22 to 30, wherein X4 is W in the amino acid sequence.

35. The composition according to any one of claims 22 to 30, wherein X5 is R in the amino acid sequence.

36. The composition according to any one of claims 22 to 30, wherein X6 is R or N in the amino acid sequence.

37. The composition according to any one of claims 22 to 30, wherein X7 is M or L in the amino acid sequence.

38. The composition according to any one of claims 22 to 30, wherein X8 is R or K in the amino acid sequence.

39. The composition according to any one of claims 22 to 30, wherein X9 is K in the amino acid sequence.

40. The composition according to any one of claims 22 to 30, wherein X1 is S in the amino acid sequence.

41. The composition according to any one of claims 22 to 30, wherein X3 is Q in the amino acid sequence.

42. The composition according to any one of claims 22 to 30, wherein X7 is M in the amino acid sequence.

43. The composition according to any one of claims 22 to 30, wherein X8 is R in the amino acid sequence.

44. The composition according to any one of claims 22 to 30, further comprising a residue X0 on the N-terminal side of the residue X1.

45. The composition according to any one of claims 22 to 30, wherein X0 is A.

46. The composition according to any one of claims 22 to 30, further comprising, on the C-terminal side of the residue X9, an amino acid sequence of:
X10-X11-X12-P-X13-X14-X15-C-X16-X17-X18-X19-X20
wherein
X10 is V, L, T or A;
X11 is G, R or N;
X12 is G, A or V;
X13 is S, P, I or L;
X14 is V, I, L or M;
X15 is S, T, F or R;
X16 is I, V, T or A;
X17 is R or K;
X18 is R or K;
X19 is A, T, D, S or Y; and
X20 is S or F.

47. The composition according to any one of claims 22 to 30, wherein the peptide contains any one amino acid sequence selected from the group consisting of amino acid sequences shown as SEQ ID NOS: 6 to 16.


### Sequence Listing

SEQUENCE LISTING

<110>  NRL Pharma
       Keio University
       Hiroshi Kawakami

<120>  Leukocyte Extracellular Trap Inhibitor

<130>  G1239WO

<150>  JP 2014-207415
<151>  2014-10-08

<160>  16

<170>  PatentIn version 3.5

<210>  1
<211>  666
<212>  PRT
<213>  Homo sapiens

<400>  1

Met Arg Lys Val Arg Gly Pro Pro Val Ser Cys Ile Lys Arg Asp Ser
1               5                   10                  15

Pro Ile Gln Cys Ile Gln Ala Ile Ala Glu Asn Arg Ala Asp Ala Val
            20                  25                  30

Thr Leu Asp Gly Gly Phe Ile Tyr Glu Ala Gly Leu Ala Pro Tyr Lys
        35                  40                  45

Leu Arg Pro Val Ala Ala Glu Val Tyr Gly Thr Glu Arg Gln Pro Arg
    50                  55                  60

Thr His Tyr Tyr Ala Val Ala Val Val Lys Lys Gly Gly Ser Phe Gln
65                  70                  75                  80

Leu Asn Glu Leu Gln Gly Leu Lys Ser Cys His Thr Gly Leu Arg Arg
                85                  90                  95

Thr Ala Gly Trp Asn Val Pro Ile Gly Thr Leu Arg Pro Phe Leu Asn
            100                 105                 110

Trp Thr Gly Pro Pro Glu Pro Ile Glu Ala Ala Val Ala Arg Phe Phe
        115                 120                 125

Ser Ala Ser Cys Val Pro Gly Ala Asp Lys Gly Gln Phe Pro Asn Leu
    130                 135                 140

Cys Arg Leu Cys Ala Gly Thr Gly Glu Asn Lys Cys Ala Phe Ser Ser
145                 150                 155                 160

Gln Glu Pro Tyr Phe Ser Tyr Ser Gly Ala Phe Lys Cys Leu Arg Asp
                165                 170                 175

Gly Ala Gly Asp Val Ala Phe Ile Arg Glu Ser Thr Val Phe Glu Asp
            180                 185                 190

Leu Ser Asp Glu Ala Glu Arg Asp Glu Tyr Glu Leu Leu Cys Pro Asp
        195                 200                 205

Asn Thr Arg Lys Pro Val Asp Lys Phe Lys Asp Cys His Leu Ala Arg
    210                 215                 220

Val Pro Ser His Ala Val Val Ala Arg Ser Val Asn Gly Lys Glu Asp
225                 230                 235                 240

Ala Ile Trp Asn Leu Leu Arg Gln Ala Gln Glu Lys Phe Gly Lys Asp
                245                 250                 255

Lys Ser Pro Lys Phe Gln Leu Phe Gly Ser Pro Ser Gly Gln Lys Asp
            260                 265                 270

Leu Leu Phe Lys Asp Ser Ala Ile Gly Phe Ser Arg Val Pro Pro Arg
        275                 280                 285

Ile Asp Ser Gly Leu Tyr Leu Gly Ser Gly Tyr Phe Thr Ala Ile Gln
    290                 295                 300

Asn Leu Arg Lys Ser Glu Glu Glu Val Ala Ala Arg Arg Ala Arg Val
305                 310                 315                 320

Val Trp Cys Ala Val Gly Glu Gln Glu Leu Arg Lys Cys Asn Gln Trp
                325                 330                 335

Ser Gly Leu Ser Glu Gly Ser Val Thr Cys Ser Ser Ala Ser Thr Thr
            340                 345                 350

Glu Asp Cys Ile Ala Leu Val Leu Lys Gly Glu Ala Asp Ala Met Ser
        355                 360                 365

Leu Asp Gly Gly Tyr Val Tyr Thr Ala Gly Lys Cys Gly Leu Val Pro
    370                 375                 380

Val Leu Ala Glu Asn Tyr Lys Ser Gln Gln Ser Ser Asp Pro Asp Pro
385                 390                 395                 400

Asn Cys Val Asp Arg Pro Val Glu Gly Tyr Leu Ala Val Ala Val Val
                405                 410                 415

Arg Arg Ser Asp Thr Ser Leu Thr Trp Asn Ser Val Lys Gly Lys Lys
        420                 425                 430

Ser Cys His Thr Ala Val Asp Arg Thr Ala Gly Trp Asn Ile Pro Met
    435                 440                 445

Gly Leu Leu Phe Asn Gln Thr Gly Ser Cys Lys Phe Asp Glu Tyr Phe
    450                 455                 460

Ser Gln Ser Cys Ala Pro Gly Ser Asp Pro Arg Ser Asn Leu Cys Ala
465                 470                 475                 480

Leu Cys Ile Gly Asp Glu Gln Gly Glu Asn Lys Cys Val Pro Asn Ser
                485                 490                 495

Asn Glu Arg Tyr Tyr Gly Tyr Thr Gly Ala Phe Arg Cys Leu Ala Glu
        500                 505                 510

Asn Ala Gly Asp Val Ala Phe Val Lys Asp Val Thr Val Leu Gln Asn
    515                 520                 525

Thr Asp Gly Asn Asn Asn Glu Ala Trp Ala Lys Asp Leu Lys Leu Ala
    530                 535                 540

Asp Phe Ala Leu Leu Cys Leu Asp Gly Lys Arg Lys Pro Val Thr Glu
545                 550                 555                 560

Ala Arg Ser Cys His Leu Ala Met Ala Pro Asn His Ala Val Val Ser
                565                 570                 575

Arg Met Asp Lys Val Glu Arg Leu Lys Gln Val Leu Leu His Gln Gln
        580                 585                 590

Ala Lys Phe Gly Arg Asn Gly Ser Asp Cys Pro Asp Lys Phe Cys Leu
    595                 600                 605

Phe Gln Ser Glu Thr Lys Asn Leu Leu Phe Asn Asp Asn Thr Glu Cys
    610                 615                 620

Leu Ala Arg Leu His Gly Lys Thr Thr Tyr Glu Lys Tyr Leu Gly Pro
625                 630                 635                 640

Gln Tyr Val Ala Gly Ile Thr Asn Leu Lys Lys Cys Ser Thr Ser Pro
                645                 650                 655

Leu Leu Glu Ala Cys Glu Phe Leu Arg Lys

660                 665

<210>  2
<211>  708
<212>  PRT
<213>  Bos taurus

<400>  2

Met Lys Leu Phe Val Pro Ala Leu Leu Ser Leu Gly Ala Leu Gly Leu
1               5                   10                  15

Cys Leu Ala Ala Pro Arg Lys Asn Val Arg Trp Cys Thr Ile Ser Gln
            20                  25                  30

Pro Glu Trp Phe Lys Cys Arg Arg Trp Gln Trp Arg Met Lys Lys Leu
        35                  40                  45

Gly Ala Pro Ser Ile Thr Cys Val Arg Arg Ala Phe Ala Leu Glu Cys
    50                  55                  60

Ile Arg Ala Ile Ala Glu Lys Lys Ala Asp Ala Val Thr Leu Asp Gly
65                  70                  75                  80

Gly Met Val Phe Glu Ala Gly Arg Asp Pro Tyr Lys Leu Arg Pro Val
                85                  90                  95

Ala Ala Glu Ile Tyr Gly Thr Lys Glu Ser Pro Gln Thr His Tyr Tyr
            100                 105                 110

Ala Val Ala Val Val Lys Lys Gly Ser Asn Phe Gln Leu Asp Gln Leu
        115                 120                 125

Gln Gly Arg Lys Ser Cys His Thr Gly Leu Gly Arg Ser Ala Gly Trp
    130                 135                 140

Ile Ile Pro Met Gly Ile Leu Arg Pro Tyr Leu Ser Trp Thr Glu Ser
145                 150                 155                 160

Leu Glu Pro Leu Gln Gly Ala Val Ala Lys Phe Phe Ser Ala Ser Cys
                165                 170                 175

Val Pro Cys Ile Asp Arg Gln Ala Tyr Pro Asn Leu Cys Gln Leu Cys
            180                 185                 190

Lys Gly Glu Gly Glu Asn Gln Cys Ala Cys Ser Ser Arg Glu Pro Tyr
        195                 200                 205

Phe Gly Tyr Ser Gly Ala Phe Lys Cys Leu Gln Asp Gly Ala Gly Asp

210                 215                 220

Val Ala Phe Val Lys Glu Thr Thr Val Phe Glu Asn Leu Pro Glu Lys
225                 230                 235                 240

Ala Asp Arg Asp Gln Tyr Glu Leu Leu Cys Leu Asn Asn Ser Arg Ala
                245                 250                 255

Pro Val Asp Ala Phe Lys Glu Cys His Leu Ala Gln Val Pro Ser His
            260                 265                 270

Ala Val Val Ala Arg Ser Val Asp Gly Lys Glu Asp Leu Ile Trp Lys
        275                 280                 285

Leu Leu Ser Lys Ala Gln Glu Lys Phe Gly Lys Asn Lys Ser Arg Ser
    290                 295                 300

Phe Gln Leu Phe Gly Ser Pro Pro Gly Gln Arg Asp Leu Leu Phe Lys
305                 310                 315                 320

Asp Ser Ala Leu Gly Phe Leu Arg Ile Pro Ser Lys Val Asp Ser Ala
                325                 330                 335

Leu Tyr Leu Gly Ser Arg Tyr Leu Thr Thr Leu Lys Asn Leu Arg Glu
            340                 345                 350

Thr Ala Glu Glu Val Lys Ala Arg Tyr Thr Arg Val Val Trp Cys Ala
        355                 360                 365

Val Gly Pro Glu Glu Gln Lys Lys Cys Gln Gln Trp Ser Gln Gln Ser
    370                 375                 380

Gly Gln Asn Val Thr Cys Ala Thr Ala Ser Thr Thr Asp Asp Cys Ile
385                 390                 395                 400

Val Leu Val Leu Lys Gly Glu Ala Asp Ala Leu Asn Leu Asp Gly Gly
                405                 410                 415

Tyr Ile Tyr Thr Ala Gly Lys Cys Gly Leu Val Pro Val Leu Ala Glu
            420                 425                 430

Asn Arg Lys Ser Ser Lys His Ser Ser Leu Asp Cys Val Leu Arg Pro
        435                 440                 445

Thr Glu Gly Tyr Leu Ala Val Ala Val Val Lys Lys Ala Asn Glu Gly
    450                 455                 460

Leu Thr Trp Asn Ser Leu Lys Asp Lys Lys Ser Cys His Thr Ala Val
465                 470                 475                 480

Asp Arg Thr Ala Gly Trp Asn Ile Pro Met Gly Leu Ile Val Asn Gln
                485                 490                 495

Thr Gly Ser Cys Ala Phe Asp Glu Phe Phe Ser Gln Ser Cys Ala Pro
            500                 505                 510

Gly Ala Asp Pro Lys Ser Arg Leu Cys Ala Leu Cys Ala Gly Asp Asp
        515                 520                 525

Gln Gly Leu Asp Lys Cys Val Pro Asn Ser Lys Glu Lys Tyr Tyr Gly
    530                 535                 540

Tyr Thr Gly Ala Phe Arg Cys Leu Ala Glu Asp Val Gly Asp Val Ala
545                 550                 555                 560

Phe Val Lys Asn Asp Thr Val Trp Glu Asn Thr Asn Gly Glu Ser Thr
                565                 570                 575

Ala Asp Trp Ala Lys Asn Leu Asn Arg Glu Asp Phe Arg Leu Leu Cys
            580                 585                 590

Leu Asp Gly Thr Arg Lys Pro Val Thr Glu Ala Gln Ser Cys His Leu
        595                 600                 605

Ala Val Ala Pro Asn His Ala Val Val Ser Arg Ser Asp Arg Ala Ala
    610                 615                 620

His Val Lys Gln Val Leu Leu His Gln Gln Ala Leu Phe Gly Lys Asn
625                 630                 635                 640

Gly Lys Asn Cys Pro Asp Lys Phe Cys Leu Phe Lys Ser Glu Thr Lys
                645                 650                 655

Asn Leu Leu Phe Asn Asp Asn Thr Glu Cys Leu Ala Lys Leu Gly Gly
            660                 665                 670

Arg Pro Thr Tyr Glu Glu Tyr Leu Gly Thr Glu Tyr Val Thr Ala Ile
        675                 680                 685

Ala Asn Leu Lys Lys Cys Ser Thr Ser Pro Leu Leu Glu Ala Cys Ala
    690                 695                 700

Phe Leu Thr Arg
705

<210>  3
<211>  708
<212>  PRT
<213>  Ovis aries

<400>  3

Met Lys Leu Phe Val Pro Ala Leu Leu Ser Leu Gly Ala Leu Gly Leu
1               5                   10                  15

Cys Leu Ala Ala Pro Arg Lys Asn Val Arg Trp Cys Ala Ile Ser Pro
            20                  25                  30

Pro Glu Gly Ser Lys Cys Tyr Gln Trp Gln Arg Arg Met Arg Lys Leu
        35                  40                  45

Gly Ala Pro Ser Ile Thr Cys Val Arg Arg Thr Ser Ala Leu Glu Cys
    50                  55                  60

Ile Arg Ala Ile Ala Gly Lys Lys Ala Asp Ala Val Thr Leu Asp Ser
65                  70                  75                  80

Gly Met Val Phe Glu Ala Gly Leu Asp Pro Tyr Lys Leu Arg Pro Val
                85                  90                  95

Ala Ala Glu Ile Tyr Gly Thr Glu Lys Ser Pro Gln Thr His Tyr Tyr
            100                 105                 110

Ala Val Ala Val Val Lys Lys Gly Ser Asn Phe Gln Leu Asp Gln Leu
        115                 120                 125

Gln Gly Gln Lys Ser Cys His Met Gly Leu Gly Arg Ser Ala Gly Trp
    130                 135                 140

Asn Ile Pro Met Gly Ile Leu Arg Pro Phe Leu Ser Trp Thr Glu Ser
145                 150                 155                 160

Ala Glu Pro Leu Gln Gly Ala Val Ala Arg Phe Phe Ser Ala Ser Cys
                165                 170                 175

Val Pro Cys Val Asp Gly Lys Ala Tyr Pro Asn Leu Cys Gln Leu Cys
            180                 185                 190

Lys Gly Val Gly Glu Asn Lys Cys Ala Cys Ser Ser Gln Glu Pro Tyr
        195                 200                 205

Phe Gly Tyr Ser Gly Ala Phe Lys Cys Leu Gln Asp Gly Ala Gly Asp
    210                 215                 220

Val Ala Phe Val Lys Glu Thr Thr Val Phe Glu Asn Leu Pro Glu Lys
225                 230                 235                 240

Ala Asp Arg Asp Gln Tyr Glu Leu Leu Cys Leu Asn Asn Thr Arg Ala
                245                 250                 255

Pro Val Asp Ala Phe Lys Glu Cys His Leu Ala Gln Val Pro Ser His
            260                 265                 270

Ala Val Val Ala Arg Ser Val Asp Gly Lys Glu Asn Leu Ile Trp Glu
        275                 280                 285

Leu Leu Arg Lys Ala Gln Glu Lys Phe Gly Lys Asn Lys Ser Gln Arg
    290                 295                 300

Phe Gln Leu Phe Gly Ser Pro Gln Gly Gln Lys Asp Leu Leu Phe Lys
305                 310                 315                 320

Asp Ser Ala Leu Gly Phe Val Arg Ile Pro Ser Lys Val Asp Ser Ala
                325                 330                 335

Leu Tyr Leu Gly Ser Arg Tyr Leu Thr Ala Leu Lys Asn Leu Arg Glu
            340                 345                 350

Thr Ala Glu Glu Val Lys Ala Arg Cys Thr Arg Val Val Trp Cys Ala
        355                 360                 365

Val Gly Pro Glu Glu His Ser Lys Cys Gln Gln Trp Ser Glu Gln Ser
    370                 375                 380

Gly Gln Asn Val Thr Cys Ala Met Ala Ser Thr Thr Asp Asp Cys Ile
385                 390                 395                 400

Ala Leu Val Leu Lys Gly Glu Ala Asp Ala Leu Ser Leu Asp Gly Gly
                405                 410                 415

Tyr Ile Tyr Thr Ala Gly Lys Cys Gly Leu Val Pro Val Met Ala Glu
            420                 425                 430

Asn Arg Glu Ser Ser Lys Tyr Ser Ser Leu Asp Cys Val Leu Arg Pro
        435                 440                 445

Thr Glu Gly Tyr Leu Ala Val Ala Val Val Lys Lys Ala Asn Glu Gly
    450                 455                 460

Leu Thr Trp Asn Ser Leu Lys Gly Lys Lys Ser Cys His Thr Ala Val
465                 470                 475                 480

Asp Arg Thr Ala Gly Trp Asn Ile Pro Met Gly Leu Ile Ala Asn Gln
                485                 490                 495

Thr Gly Ser Cys Ala Phe Asp Glu Phe Phe Ser Gln Ser Cys Ala Pro
            500                 505                 510

Gly Ala Asp Pro Lys Ser Ser Leu Cys Ala Leu Cys Ala Gly Asp Asp
        515                 520                 525

Gln Gly Leu Asn Lys Cys Val Pro Asn Ser Lys Glu Lys Tyr Tyr Gly
    530                 535                 540

Tyr Thr Gly Ala Phe Arg Cys Leu Ala Glu Asp Val Gly Asp Val Ala
545                 550                 555                 560

Phe Val Lys Asn Asp Thr Val Trp Glu Asn Thr Asn Gly Glu Ser Ser
                565                 570                 575

Ala Asp Trp Ala Lys Asn Leu Asn Arg Glu Asp Phe Arg Leu Leu Cys
            580                 585                 590

Leu Asp Gly Thr Thr Lys Pro Val Thr Glu Ala Gln Ser Cys Tyr Leu
        595                 600                 605

Ala Val Ala Pro Asn His Ala Val Val Ser Arg Ser Asp Arg Ala Ala
    610                 615                 620

His Val Glu Gln Val Leu Leu His Gln Gln Ala Leu Phe Gly Lys Asn
625                 630                 635                 640

Gly Lys Asn Cys Pro Asp Gln Phe Cys Leu Phe Lys Ser Glu Thr Lys
                645                 650                 655

Asn Leu Leu Phe Asn Asp Asn Thr Glu Cys Leu Ala Lys Leu Gly Gly
            660                 665                 670

Arg Pro Thr Tyr Glu Lys Tyr Leu Gly Thr Glu Tyr Val Thr Ala Ile
        675                 680                 685

Ala Asn Leu Lys Lys Cys Ser Thr Ser Pro Leu Leu Glu Ala Cys Ala
    690                 695                 700

Phe Leu Thr Arg
705

<210>  4

<211>  708
<212>  PRT
<213>  Capra hircus

<400>  4

Met Lys Leu Phe Val Pro Ala Leu Leu Ser Leu Gly Ala Leu Gly Leu
1               5                   10                  15

Cys Leu Ala Ala Pro Arg Lys Asn Val Arg Trp Cys Ala Ile Ser Leu
            20                  25                  30

Pro Glu Trp Ser Lys Cys Tyr Gln Trp Gln Arg Arg Met Arg Lys Leu
        35                  40                  45

Gly Ala Pro Ser Ile Thr Cys Ile Arg Arg Thr Ser Ala Leu Glu Cys
    50                  55                  60

Ile Arg Ala Ile Ala Gly Lys Asn Ala Asp Ala Val Thr Leu Asp Ser
65                  70                  75                  80

Gly Met Val Phe Glu Ala Gly Leu Asp Pro Tyr Lys Leu Arg Pro Val
                85                  90                  95

Ala Ala Glu Ile Tyr Gly Thr Glu Lys Ser Pro Gln Thr His Tyr Tyr
            100                 105                 110

Ala Val Ala Val Val Lys Lys Gly Ser Asn Phe Gln Leu Asp Gln Leu
        115                 120                 125

Gln Gly Gln Lys Ser Cys His Met Gly Leu Gly Arg Ser Ala Gly Trp
    130                 135                 140

Asn Ile Pro Val Gly Ile Leu Arg Pro Phe Leu Ser Trp Thr Glu Ser
145                 150                 155                 160

Ala Glu Pro Leu Gln Gly Ala Val Ala Arg Phe Phe Ser Ala Ser Cys
                165                 170                 175

Val Pro Cys Val Asp Gly Lys Ala Tyr Pro Asn Leu Cys Gln Leu Cys
            180                 185                 190

Lys Gly Val Gly Glu Asn Lys Cys Ala Cys Ser Ser Gln Glu Pro Tyr
        195                 200                 205

Phe Gly Tyr Ser Gly Ala Phe Lys Cys Leu Gln Asp Gly Ala Gly Asp
    210                 215                 220

Val Ala Phe Val Lys Glu Thr Thr Val Phe Glu Asn Leu Pro Glu Lys

225                 230                 235                 240

Ala Asp Arg Asp Gln Tyr Glu Leu Leu Cys Leu Asn Asn Thr Arg Ala
                245                 250                 255

Pro Val Asp Ala Phe Lys Glu Cys His Leu Ala Gln Val Pro Ser His
            260                 265                 270

Ala Val Val Ala Arg Ser Val Asp Gly Lys Glu Asn Leu Ile Trp Glu
        275                 280                 285

Leu Leu Arg Lys Ala Gln Glu Lys Phe Gly Lys Asn Lys Ser Gln Ser
    290                 295                 300

Phe Gln Leu Phe Gly Ser Pro Glu Gly Arg Arg Asp Leu Leu Phe Lys
305                 310                 315                 320

Asp Ser Ala Leu Gly Phe Val Arg Ile Pro Ser Lys Val Asp Ser Ala
                325                 330                 335

Leu Tyr Leu Gly Ser Arg Tyr Leu Thr Ala Leu Lys Asn Leu Arg Glu
            340                 345                 350

Thr Ala Glu Glu Leu Lys Ala Arg Cys Thr Arg Val Val Trp Cys Ala
        355                 360                 365

Val Gly Pro Glu Glu Gln Ser Lys Cys Gln Gln Trp Ser Glu Gln Ser
    370                 375                 380

Gly Gln Asn Val Thr Cys Ala Thr Ala Ser Thr Thr Asp Asp Cys Ile
385                 390                 395                 400

Ala Leu Val Leu Lys Gly Glu Ala Asp Ala Leu Ser Leu Asp Gly Gly
                405                 410                 415

Tyr Ile Tyr Thr Ala Gly Lys Cys Gly Leu Val Pro Val Met Ala Glu
            420                 425                 430

Asn Arg Lys Ser Ser Lys Tyr Ser Ser Leu Asp Cys Val Leu Arg Pro
        435                 440                 445

Thr Glu Gly Tyr Leu Ala Val Ala Val Val Lys Lys Ala Asn Glu Gly
    450                 455                 460

Leu Thr Trp Asn Ser Leu Lys Gly Lys Lys Ser Cys His Thr Ala Val
465                 470                 475                 480

Asp Arg Thr Ala Gly Trp Asn Ile Pro Met Gly Leu Ile Ala Asn Gln
                485                 490                 495

Thr Gly Ser Cys Ala Phe Asp Glu Phe Phe Ser Gln Ser Cys Ala Pro
            500                 505                 510

Gly Ala Asp Pro Lys Ser Ser Leu Cys Ala Leu Cys Ala Gly Asp Asp
        515                 520                 525

Gln Gly Leu Asp Lys Cys Val Pro Asn Ser Lys Glu Lys Tyr Tyr Gly
    530                 535                 540

Tyr Thr Gly Ala Phe Arg Cys Leu Ala Glu Asp Val Gly Asp Val Ala
545                 550                 555                 560

Phe Val Lys Asn Asp Thr Val Trp Glu Asn Thr Asn Gly Glu Ser Ser
                565                 570                 575

Ala Asp Trp Ala Lys Asn Leu Asn Arg Glu Asp Phe Arg Leu Leu Cys
            580                 585                 590

Leu Asp Gly Thr Thr Lys Pro Val Thr Glu Ala Gln Ser Cys Tyr Leu
        595                 600                 605

Ala Val Ala Pro Asn His Ala Val Val Ser Arg Ser Asp Arg Ala Ala
    610                 615                 620

His Val Glu Gln Val Leu Leu His Gln Gln Ala Leu Phe Gly Lys Asn
625                 630                 635                 640

Gly Lys Asn Cys Pro Asp Gln Phe Cys Leu Phe Lys Ser Glu Thr Lys
                645                 650                 655

Asn Leu Leu Phe Asn Asp Asn Thr Glu Cys Leu Ala Lys Leu Gly Gly
            660                 665                 670

Arg Pro Thr Tyr Glu Lys Tyr Leu Gly Thr Glu Tyr Val Thr Ala Ile
        675                 680                 685

Ala Asn Leu Lys Lys Cys Ser Thr Ser Pro Leu Leu Glu Ala Cys Ala
    690                 695                 700

Phe Leu Thr Arg
705

<210>  5
<211>  695
<212>  PRT

<213>  Equus caballus

<400>  5

Leu Gly Leu Cys Leu Ala Ala Pro Arg Lys Ser Val Arg Trp Cys Thr
1               5                   10                  15

Ile Ser Pro Ala Glu Ala Ala Lys Cys Ala Lys Phe Gln Arg Asn Met
            20                  25                  30

Lys Lys Val Arg Gly Pro Ser Val Ser Cys Ile Arg Lys Thr Ser Ser
        35                  40                  45

Phe Glu Cys Ile Gln Ala Ile Ala Ala Asn Lys Ala Asp Ala Val Thr
    50                  55                  60

Leu Asp Gly Gly Leu Val Tyr Glu Ala Gly Leu His Pro Tyr Lys Leu
65                  70                  75                  80

Arg Pro Val Ala Ala Glu Val Tyr Gln Thr Arg Gly Lys Pro Gln Thr
                85                  90                  95

Arg Tyr Tyr Ala Val Ala Val Val Lys Lys Gly Ser Gly Phe Gln Leu
            100                 105                 110

Asn Gln Leu Gln Gly Val Lys Ser Cys His Thr Gly Leu Gly Arg Ser
        115                 120                 125

Ala Gly Trp Asn Ile Pro Ile Gly Thr Leu Arg Pro Tyr Leu Asn Trp
    130                 135                 140

Thr Gly Pro Pro Glu Pro Leu Gln Lys Ala Val Ala Asn Phe Phe Ser
145                 150                 155                 160

Ala Ser Cys Val Pro Cys Ala Asp Gly Lys Gln Tyr Pro Asn Leu Cys
                165                 170                 175

Arg Leu Cys Ala Gly Thr Glu Ala Asp Lys Cys Ala Cys Ser Ser Gln
            180                 185                 190

Glu Pro Tyr Phe Gly Tyr Ser Gly Ala Phe Lys Cys Leu Glu Asn Gly
        195                 200                 205

Ala Gly Asp Val Ala Phe Val Lys Asp Ser Thr Val Phe Glu Asn Leu
    210                 215                 220

Pro Asp Glu Ala Asp Arg Asp Lys Tyr Glu Leu Leu Cys Pro Asp Asn
225                 230                 235                 240

Thr Arg Lys Pro Val Asp Ala Phe Lys Glu Cys His Leu Ala Arg Val
                245                 250                 255

Pro Ser His Ala Val Val Ala Arg Ser Val Asp Gly Arg Glu Asp Leu
            260                 265                 270

Ile Trp Arg Leu Leu His Arg Ala Gln Glu Glu Phe Gly Arg Asn Lys
        275                 280                 285

Ser Ser Ala Phe Gln Leu Phe Lys Ser Thr Pro Glu Asn Lys Asp Leu
    290                 295                 300

Leu Phe Lys Asp Ser Ala Leu Gly Phe Val Arg Ile Pro Ser Gln Ile
305                 310                 315                 320

Asp Ser Gly Leu Tyr Leu Gly Ala Asn Tyr Leu Thr Ala Thr Gln Asn
                325                 330                 335

Leu Arg Glu Thr Ala Ala Glu Val Ala Ala Arg Arg Glu Arg Val Val
            340                 345                 350

Trp Cys Ala Val Gly Pro Glu Glu Glu Arg Lys Cys Lys Gln Trp Ser
        355                 360                 365

Asp Val Ser Asn Arg Lys Val Ala Cys Ala Ser Ala Ser Thr Thr Glu
    370                 375                 380

Glu Cys Ile Ala Leu Val Leu Lys Gly Glu Ala Asp Ala Leu Asn Leu
385                 390                 395                 400

Asp Gly Gly Phe Ile Tyr Val Ala Gly Lys Cys Gly Leu Val Pro Val
                405                 410                 415

Leu Ala Glu Asn Gln Lys Ser Gln Asn Ser Asn Ala Pro Asp Cys Val
            420                 425                 430

His Arg Pro Pro Glu Gly Tyr Leu Ala Val Ala Val Val Arg Lys Ser
        435                 440                 445

Asp Ala Asp Leu Thr Trp Asn Ser Leu Ser Gly Lys Lys Ser Cys His
    450                 455                 460

Thr Gly Val Gly Arg Thr Ala Ala Trp Asn Ile Pro Met Gly Leu Leu
465                 470                 475                 480

Phe Asn Gln Thr Gly Ser Cys Lys Phe Asp Lys Phe Phe Ser Gln Ser
                485                 490                 495

Cys Ala Pro Gly Ala Asp Pro Gln Ser Ser Leu Cys Ala Leu Cys Val
            500                 505                 510

Gly Asn Asn Glu Asn Glu Asn Lys Cys Met Pro Asn Ser Glu Glu Arg
        515                 520                 525

Tyr Tyr Gly Tyr Thr Gly Ala Phe Arg Cys Leu Ala Glu Lys Ala Gly
    530                 535                 540

Asp Val Ala Phe Val Lys Asp Val Thr Val Leu Gln Asn Thr Asp Gly
545                 550                 555                 560

Lys Asn Ser Glu Pro Trp Ala Lys Asp Leu Lys Gln Glu Asp Phe Glu
                565                 570                 575

Leu Leu Cys Leu Asp Gly Thr Arg Lys Pro Val Ala Glu Ala Glu Ser
            580                 585                 590

Cys His Leu Ala Arg Ala Pro Asn His Ala Val Val Ser Gln Ser Asp
        595                 600                 605

Arg Ala Gln His Leu Lys Lys Val Leu Phe Leu Gln Gln Asp Gln Phe
    610                 615                 620

Gly Gly Asn Gly Pro Asp Cys Pro Gly Lys Phe Cys Leu Phe Lys Ser
625                 630                 635                 640

Glu Thr Lys Asn Leu Leu Phe Asn Asp Asn Thr Glu Cys Leu Ala Glu
                645                 650                 655

Leu Gln Gly Lys Thr Thr Tyr Glu Gln Tyr Leu Gly Ser Glu Tyr Val
            660                 665                 670

Thr Ser Ile Thr Asn Leu Arg Arg Cys Ser Ser Ser Pro Leu Leu Glu
        675                 680                 685

Ala Cys Ala Phe Leu Arg Ala
    690                 695

<210> 6
<211> 26
<212> PRT
<213> Bos taurus

<400> 6

Met Phe Lys Cys Arg Arg Trp Gln Trp Arg Met Lys Lys Leu Gly Ala
1               5                   10                  15

Pro Ser Ile Thr Cys Val Arg Arg Ala Phe
            20                  25

<210>  7
<211>  49
<212>  PRT
<213>  Homo sapiens

<400>  7

Gly Arg Arg Arg Arg Ser Val Gln Trp Cys Ala Val Ser Gln Pro Glu
1               5                   10                  15

Ala Thr Lys Cys Phe Gln Trp Gln Arg Asn Met Arg Lys Val Arg Gly
            20                  25                  30

Pro Pro Val Ser Cys Ile Lys Arg Asp Ser Pro Ile Gln Cys Ile Gln
        35                  40                  45

Ala

<210>  8
<211>  26
<212>  PRT
<213>  Macaca cyclopis

<400>  8

Ala Thr Lys Cys Ser Gln Trp Gln Arg Asn Leu Arg Arg Val Arg Gly
1               5                   10                  15

Pro Pro Val Ser Cys Ile Lys Arg Ala Ser
            20                  25

<210>  9
<211>  26
<212>  PRT
<213>  Capra hircus

<400>  9

Trp Ser Lys Cys Tyr Gln Trp Gln Arg Arg Met Arg Lys Leu Gly Ala
1               5                   10                  15

Pro Ser Ile Thr Cys Ile Arg Arg Thr Ser
            20                  25

<210>  10
<211>  26
<212>  PRT
<213>  Ovis aries

<400>  10

Gly Ser Lys Cys Tyr Gln Trp Gln Arg Arg Met Arg Lys Leu Gly Ala
1               5                   10                  15

Pro Ser Ile Thr Cys Val Arg Arg Thr Ser
            20                  25

<210>  11
<211>  26
<212>  PRT
<213>  Equus caballus

<400>  11

Ala Ala Lys Cys Ala Lys Phe Gln Arg Asn Met Lys Lys Val Arg Gly
1               5                   10                  15

Pro Ser Val Ser Cys Ile Arg Lys Thr Ser
            20                  25

<210>  12
<211>  26
<212>  PRT
<213>  Camelus dromedarius

<400>  12

Ser Ser Lys Cys Ala Gln Trp Gln Arg Arg Met Lys Lys Val Arg Gly
1               5                   10                  15

Pro Ser Val Thr Cys Val Lys Lys Thr Ser
            20                  25

<210>  13
<211>  26
<212>  PRT
<213>  Canis lupus familiaris

<400>  13

Ala Lys Lys Cys Ser Lys Phe Gln Val Asn Met Lys Lys Val Gly Gly
1               5                   10                  15

Pro Ile Val Ser Cys Thr Arg Lys Ala Ser
            20                  25

<210>  14
<211>  26
<212>  PRT
<213>  Mus musculus

<400>  14

Glu Glu Lys Cys Leu Arg Trp Gln Asn Glu Met Arg Lys Val Gly Gly
1               5                   10                  15

Pro Pro Leu Ser Cys Val Lys Lys Ser Ser
            20                  25

<210>  15
<211>  26
<212>  PRT
<213>  Sus scrofa domesticus

<400>  15

Tyr Ser Lys Cys Arg Gln Trp Gln Ser Lys Ile Arg Arg Thr Asn Pro
1               5                   10                  15

Met Phe Cys Ile Arg Arg Ala Ser Pro Thr
            20                  25

<210>  16
<211>  26
<212>  PRT
<213>  rattus norvegicus

<400>  16

Ala Gln Lys Cys Phe Met Trp Gln Glu Met Leu Asn Lys Ala Gly Val
1               5                   10                  15

Pro Lys Leu Arg Cys Ala Arg Lys Tyr Phe
            20                  25